# EUROPEAN PATENT APPLICATION

(11) **EP 1 459 759 A1**
(43) Date of publication of application: **22.09.2004**
(21) Application number: 02803523.6
(22) Date of filing: 19.11.2002
(51) Int. Cl.: A61K 38/19, A61K 38/00, A61P 1/16, A61P 1/18, A61P 5/14, A61P 7/00, A61P 9/00, A61P 13/12, A61P 17/00, A61P 19/00, A61P 21/00, A61P 25/00, A61P 43/00

(54) **DRUG MOBILIZING PLURIPOTENT STEM CELLS FROM TISSUE INTO PERIPHERAL BLOOD**

(30) Priority: 19.11.2001 JP 2001353788
(71) Applicant: KYOWA HAKKO KOGYO CO., LTD., Chiyoda-ku, Tokyo 100-8185 (JP)
(72) Inventor: SATO, Hidetaka, c/o Kyowa Hakko Kogyo Co. Ltd., Machida-shi, Tokyo 194-8533 (JP); YAMADA, Yoji, c/o Kyowa Hakko Kogyo Co. Ltd., Machida-shi, Tokyo 194-8533 (JP); ANDO, Hiroshi, c/o Kyowa Hakko Kogyo Co. Ltd., Machida-shi, Tokyo 194-8533 (JP); YOKOYAMA, Hiromi, c/o Kyowa Hakko Kogyo Co. Ltd., Machida-shi, Tokyo 194-8533 (JP); SAKURADA, Kazuhiro, c/o Kyowa Hakko Kogyo Co. Ltd., Chiyoda-ku, Tokyo 100-8185 (JP)
(74) Representative: VOSSIUS & PARTNER
(86) International application number: PCT/JP2002/012060
(87) International publication number: WO 2003/043651

(57) **Abstract**

The present invention relates to an agent which mobilizes multipotential stem cells from tissues such as bone marrow, skin and skeletal muscle to peripheral blood. The present invention further relates to a method for the treatment of cardiac insufficiency, hepatic insufficiency, renal insufficiency, neurodegenerative diseases, (such as Parkinson's disease and Alzheimer's disease), cardiovascular disturbance, cerebrovascular disturbance, spinal damage, arthritis, osteoporosis, diabetes mellitus, etc. using the agent which mobilizes multipotential stem cells from tissues such as bone marrow, skin and skeletal muscle to peripheral blood.

## Description

### Technical Field

The present invention relates to an agent which mobilizes multipotential stem cells haying an ability of differentiating into various cells existing in adult tissues such as red blood cells, white blood cells, platelets, fibroblasts, adipocytes, skeletal muscle cells, smooth muscle cells, cardiacmuscle cells, neurons, glias, oligodendrocytes, vascular endothelial cells, epidermal cells of skin, dermal cells of skin, hair follicle cells, osteocytes, osteoblasts, osteoclasts, chondrocytes, epithelial cells of trachea, alveolar cells, epithelial cells of gastrointestinal tract, epithelial cells of mouth, ameloblasts, odontoblasts, hepatocytes, Kupffer's cells, epithelial cells of gallbladder, endocrine cells of pancreas, exocrine cells of pancreas, tubular cells of kidney, renal glomeruli cells, epithelial cells of ureter, epithelial cells of urinary tract, endocrine cells of pituitary gland, thyroid cells, adrenocortical cells, adrenomedullary cells, prostatic cells, mammary gland cells, visual cells, pigment epithelial cells, corneal cells, lacrimal cells and tympanic cells to peripheral blood; an agent for the treatment by a tissue regeneration containing the agent; a method for recovering multipotential stem cells using the agent; and a method for inducing differentiation of the multipotential cells prepared by the above method to somatic cells.

### Background of the Invention

At present, organ transplantation from a brain-dead donor is carried out as a radical therapy for cardiac insufficiency, hepatic insufficiency, renal insufficiency, neurodegenerative diseases (such as Parkinson' s disease and Alzheimer' s disease), cardiovascular disturbance, cerebrovascular disturbance, spinal damage; arthritis, osteoporosis, diabetes mellitus, etc. However, due to shortage of donors, even in the United States where transplant therapy has developed, organs are available only for about 5% of patients who wish the organ transplantation [*J. Am. Med. Assoc.*, 267, 239-246 (1992)].

In addition, organ transplantation is accompanied by immunological rejection, and therefore, immunosuppressants should be administered throughout one' s life and there is risk of suffering from infectious diseases derived from donor.

In order to solve the shortage of donors, development of technique concerning heterotransplantation has been in progress.

However, the problem of immunological rejection has not been solved yet and degree of rejection is severer as compared with the homotransplantation. There is also a risk of infection of virus, etc. via the transplantation from pigs which are believed to be effective materials for heterotransplantation. In fact, in Malaysia, nearly 1,000,000 pigs were infected by Hendra virus and, as a result, fatal infection was induced to more than 70 people [*Nature,* 398, 549 (1999)].

In order to overcome the above-mentioned problems concerning homotransplantation and heterotransplantation, a technique of differentiation and induction of human tissues and cells from stem cells have been investigated.

Stem cell is a cell having both self-replicating ability and multipotential ability of being able to differentiate to various tissues. Stem cells may be roughly classified into five in view of their collected sites and they are embryonic stem cell (ES cell), fetal stem cell, adult stem cell, cord blood stem cell and placenta stem cell. ES cell (embryonic stem cell) separated from a site called internal cell mass of embryo at blastula stage and EG cell (embryonic germ cell) collected from germ line of fetus have a totipotency being able to differentiate to all cells of adults and, therefore, they have drawn public attention as materials for reconstruction of tissues [*Konnnichi no Ishoku,* 14, 542-548 (2001)]. In addition, from tissues of fetus, tissue-specific stem cells such as neural stem cell have been separated and cultured [*Proc*. *Natl. Acad. Sci*. *USA*, 97, 14720-14725 (2000)]. However, for the preparation of such stem cells derived from embryo and fetus, it is necessary to destroy the embryo and fetus, and there is an ethical problem therefore. Moreover, stem cells are not cells of the patient himself/herself and it is not easy to avoid immunological rejection as in the case of organ transplantation from a brain-dead donor. Furthermore, since stem cells of embryo and stem cells of fetus are the cells which function for generation of individuals, their properties are different from those of stem cells of adults and their affinity to adult tissues is different as well. In fact, when ES cells are transplanted to adult tissues, tumor is formed. The above shows that it is not easy to test and prove the safety for a long period for the therapeutic method by transplantation of cells derived from embryo and fetus to adults.

In the case of stem cells derived from adult tissues, therapy using the cells of the patient himself/herself is possible.

Since stem cells have a self-replicating ability, they are able to be manufactured in large quantities. Accordingly, it is possible to guarantee the safety upon transplantation by proving that the stem cells which are cultured and manufactured *in vitro* are identical to the stem cells in the tissues. Commercial products for replication of skin and cartilage have been sold already [*Tanpakushitsu Kakusan Koso,* 45, 2342-2347 (2000)].

However, the tissue-specific stem cells existing in the tissues of adults have a limited ability for division, and therefore, there is a disadvantage that availability of sufficient amount of cells is difficult. In addition, so far as the tissue-specific stem cells are used, they are able to be utilized for the therapy of such tissues only and there is no multiplicity of uses.

Recently, however, it has been clarified that multipotential stem cells which have an ability of differentiating to nearly all kinds of cells in adults are present in adult tissues (WO 01/11011, WO 01/21767, WO 01/48149).

Since such cells have a self-replicating ability showing an unlimited growth, it is also possible to manufacture the cells in large quantities. Further, unlike the ES cells derived from embryo, those cells do not form tumor even when transplanted to adult cells. It has been shown that such multipotential stem cells can be prepared from human skin, skeletal muscle and bone marrow after birth.

On the other hand, rebuilding of hematopoietic system by transplantation of hematopoietic stem cells for the purpose of treatment of various diseases has been attempted. Hematopoietic stem cells are collected from bone marrow and, collection of stem cells trombone marrow is a burden to patients. Therefore, in view of making the collection easy or making the transplantation time short, it has been attempted to mobilize the hematopoietic stem cells to peripheral blood and to collect therefrom rather than to collect the hematopoietic stem cells from bone marrow.

As a method for mobilization of the hematopoietic stem cells to peripheral blood, a method which utilizes growth factor of hematopoietic system, chemokine, cytotoxic agent, etc. have been known.

G-CSF (granulocyte colony-stimulating factor), GM-CSF (granulocyte-macrophage colony-stimulating factor), IL-7, IL-12, flt-3 ligand, stem cell factor (SCF) , thrombopoietin, etc. have been known as growth factors for hematopoietic system. IL-8, :MIP-2 (macrophage inflammatory protein-2), BB-10010 (MIP-1α) , etc. have been known as chemokines. Cyclophosphamide, etc. have been known as cytotoxic agents [*Blood*, 10, 3025-3031 (2000)].

A study group of the Columbia University found that, when CD 34-positive cells were separated from cells mobilized to human peripheral blood by G-CSF and transplanted to a cardiac infarction model, angiogenesis took place and cardiac function was improved [*Nature Medicine,* 7, 430-436 (2001), WO 2001/94420].

A study group of the New York State University found that, when Lin-negative and c-kit-positive hematopoietic stem cells in bone marrow of mice were transplanted to mice suffering from cardiac infarction, cardiac muscle and blood vessel were regenerated [*Nature*, 410, 701-705 (2001)]. The same study group also found that, when G-CSF and SCF were administered to mice before onset of cardiac infarction, regeneration of cardiac muscle and blood vessel took place in the infarction site, and the group considered that hematopoietic stem cells flew out to peripheral blood by G-CSF whereupon the infarcted cardiac muscle was regenerated [*Proc. Natl*. *Acad. Sci. USA*, 98, 10344-10349 (2001)].

### Disclosure of the Invention

Multipotential stem cells are able to regenerate various tissues. Collection of the multipotential stemcells fromskin, skeletal muscle and bone marrow of a patient involves problems such as strong pain to the patient himself/herself, tissue destruction, etc. Therefore, in order to easily conduct the collection from peripheral blood of a patient, there has been a demand for a drug which mobilizes the multipotential stem cells to peripheral blood. There has been also a demand for finding a factor which simplifies the steps of regenerative medical treatment using cells, cell collection from a patient, cell culture *in vitro* and cell transplantation to a patient, to enable tissue destruction and denaturation without the cell transplantation.

Intensive investigations have been carried out for various factors in order to solve the above-mentioned problems, and as a result, cytokine which has been believed to act on activation of immune system has been found to have an action of mobilizing the'multipotential stem cells participating in various tissue repairs to peripheral blood, and thus the present invention has been achieved.

The present invention relates to the following (1) to (40).
(1) An agent mobilizing the multipotential stem cells from tissues to peripheral blood which contains, as an active ingredient, a cytokine selected from the group consisting of a cytokine which activates monocyte or macrophage, a cytokine which is secreted from the activated monocyte or macrophage and a cytokine which is secreted from hematopoietic cells where G-CSF receptor is expressed.
(2) The agent mentioned in the above (1), wherein the cytokine is a cytokine which is selected from the group consisting of G-CSF, M-CSF, GM-CSF and IL-8.
(3) The agent mentioned in the above (1), wherein the cytokine which activates monocyte or macrophage is selected from the group consisting of M-CSF and GM-CSF.
(4) The agent mentioned in the above (1), wherein the hematopoietic cells expressing the G-CSF receptor is granulocyte or neutrophil.
(5) The agent mentioned in the above (4), wherein the cytokine secreted from neutrophil is IL-8.
(6) The agent mentioned in any of the above (1), to (5), wherein the cytokine is a chemically modified one.
(7) The agent mentioned in the above (6), wherein the cytokine is chemically modified with polyalkylene glycol.
(8) The agent mentioned in any of the above (1) to (7), wherein one or several amino acid(s) in the amino acid sequence constituting the cytokine is/are deleted, substituted, inserted or added and the cytokine has the substantially same activity.
(9) The agent mentioned in the above (1), wherein the tissue is bone marrow.
(10) The agent mentioned in the above (1), wherein the multipotential stem cell is selected from the group consisting of CD 45-negative cell, glycophorin:A-negative cell and oct 3/4-positive cell.
(11) The agent mentioned in the above (1), wherein the multipotential stem cell is selected from the group consisting of CD 45-negative and glycophorin A-negative cell, oct 3/4-positive and CD 45-negative cell, oct 3/4-positive and glycophorin A-negative cell, CD 45-negative, glycophorin A-negative and oct 3/4-positive cell and CD 45-negative and CD 34-negative cell.
(12) The agent mentioned in the above (1), wherein the multipotential stem cell is selected from the group consisting of CD 34-positive, CD 117-positive and CD 140-positive cell, CD 10-positive and CD 66e-positive cell, CD, 13-positive and CD 49b-positive cell and CD 45-negative and CD 34-positive cell.
(13) A method for the recovery of the multipotential stem cells mobilized to the peripheral blood stem cells, wherein the agent mentioned in any of the above (1) to (12) is used.
(14) A method for regeneration of tissues, wherein the multipotential stem cells obtained by the method mentioned in the above (13) is used.
(15) A method for inducing the differentiation of the multipotential stem cells mobilized to the peripheral blood stem cells to somatic cells using the agent mentioned in any of the above (1) to (12).
(16) The method mentioned in the above (15), wherein the somatic cells are those which are selected from the group consisting of red blood cells, white blood cells, platelets, fibroblasts, adipocytes, skeletal muscle cells, smooth muscle cells, cardiac muscle cells,neurons,glias,oligodendrocytes, vascular endothelial cells, epidermal cells of skin, dermal cells of skin, hair follicle cells, osteocytes, osteoblasts, osteoclasts, chondrocytes, epithelial cells of trachea, alveolar cells, epithelial cells of gastrointestinal tract, epithelial cells of mouth, ameloblasts, odontoblasts, hepatocytes, Kupffer's cells, epithelial cells of gallbladder, endocrine cells of pancreas, exocrine cells of pancreas, tubular cells of kidney, renal glomeruli cells, epithelial cells of ureter, epithelial cells of urinary tract, endocrine cells of pituitary gland, thyroid cells, adrenocortical cells, adrenomedullary cells, prostatic cells, mammary gland cells, visual cells, pigment epithelial cells, corneal cells, lacrimal cells and tympanic cells.
(17) A tissue regeneration therapeutic agent which contains, as an active ingredient, a cytokine selected from the group consisting of a cytokine which activates monocyte or macrophage, a cytokine which is secreted from the activated monocyte or macrophage and a cytokine which is secreted from hematopoietic cells where G-CSF receptor is expressed.
(18). The therapeutic agent mentioned in the above (17), wherein the cytokine is selected from the group consisting of G-CSF, M-CSF, GM-CSF and IL-8.
(19) The therapeutic agent mentioned in the above (17), wherein the cytokine which activates monocyte or macrophage is selected from the group consisting of M-CSF and GM-CSF.
(20) The therapeutic agent mentioned in the above (17), wherein the hematopoietic cells expressing the G-CSF receptor is granulocyte or neutrophil.
(21) The therapeutic agent mentioned in the above (20), wherein the cytokine secreted from neutrophil is IL-8.
(22) The therapeutic agent mentioned in any of the above (17) to (21), wherein the cytokine is a chemically modified one.
(23) The therapeutic agent mentioned in the above (22), wherein the cytokine is chemically modified with polyalkylene glycol.
(24) The therapeutic agent mentioned in any of the above (17) to (23), wherein one or several amino acid(s) in the amino acid sequence constituting the cytokine is/are deleted, substituted, inserted or added and the cytokine has the substantially same activity.
(25) The therapeutic agent mentioned in any of the above (17) to (24), wherein the tissue is selected from the group consisting of neural tissue, skin tissue, cardiovascular tissue, respiratory tissue, skeletal tissue, connective tissue, blood, immunological tissue, enteric tissue, oral tissue, hepatic tissue, pancreatic tissue, urinary tissue, endocrine gland and sensory tissue.
(26) The therapeutic agent mentioned in the above (25), wherein the neural tissue is selected from the group consisting of central nervous system and peripheral nervous system.
(27) The therapeutic agent mentioned in the above (25), wherein the skin tissue is selected from the group consisting of epidermis of skin, dermis of skin and hypodermal tissue.
(28) The therapeutic agent mentioned in the above (25), wherein the cardiovascular tissue is selected from the group consisting of heart, blood vessel of artery, blood vessel of vein, capillary vessel and lymph vessel.
(29) The therapeutic agent mentioned in the above (25), wherein the respiratory tissue is selected from the group consisting of viscous membrane of nose, airway and air cell.
(30) The therapeutic agent mentioned in the above (25), wherein the skeletal tissue is selected from the group consisting of bone, cartilage, joint, ligament and tendon.
(31) The therapeutic agent mentioned in the above (25), wherein the connective tissue is selected from the group consisting of fat tissue and fiber tissue.
(32) The therapeutic agent mentioned in the above (25), wherein the blood tissue is selected from the group consisting of red blood cell, white blood cell and platelet.
(33) The therapeutic agent mentioned in the above (25), wherein the immunological tissue is selected from the group consisting of lymphocyte, monocyte, granulocyte, thymus, lymph node and spleen.
(34) The therapeutic agent mentioned in the above (25), wherein the enteric tissue is selected from the group consisting of salivary gland, stomach, duodenum, small intestine, large intestine, rectum and anus.
(35) The therapeutic agent mentioned in the above (25), wherein the oral tissue is selected from the group consisting of mucous membrane of mouth, tooth and tongue.
(36) The therapeutic agent mentioned in the above (25), wherein the hepatic tissue is selected from the group consisting of liver and gallbladder.
(37) The therapeutic agent mentioned in the above (25), wherein the pancreatic tissue is selected from the group consisting of exocrine gland of pancreas and endocrine gland of pancreas.
(38) The therapeutic agent mentioned in the above (25), wherein the urinary tissue is selected from the group consisting of renal nephron, urinary tubule of kidney, renal medulla, ureter, bladder and urinary tract.
(39) The therapeutic agent mentioned in the above (25), wherein the endocrine gland is selected from the group consisting of pituitary gland, thyroid gland and peripheral nerve.
(40) The therapeutic agent mentioned in the above (25), wherein the sensory tissue is selected from the group consisting of gustatory bud, olfactory epithelium, retina, cornea, crystalline lens, labyrinth and peripheral nerve system.

The present invention will be described in detail below.

### 1. Agents which mobilize the multipotential stem cells from tissues to peripheral blood

The present invention relates to an agent mobilizing the multipotential stemcells fromtissues to peripheral blood which contains, as an active ingredient, a cytokine such as cytokine which activates granulocyte, monocyte or macrophage, a cytokine which is secreted from the activated monocyte or macrophage and a cytokine which is secreted from hematopoietic cells where G-CSF receptor is expressed.

Any cytokine having the above-mentioned property can be used as an agent of the present invention.

For example, M-CSF, GM-CSF, etc. may be exemplified as cytokine which activates granulocyte, monocyte and macrophage; G-CSF, GM-CSF, etc. may be exemplified as cytokine which is secreted from the activated macrophage; and IL-8, etc. may be exemplified as cytokine which is secreted from neutrophil.

Although metalloproteinase gelatinase B (hereinafter, abbreviated as MMP-9) is not cytokine, it is adopted as a substance which is identical with the above-mentioned cytokine in the present invention since it has the same activity as the cytokine secreted from neutrophil.

Cytokine where the above-mentioned cytokine is chemically modified can also be used as the drug of the present invention. Examples of a method for the chemical modification are modification by polyalkylene glycol (WO 98/55500), modification by albumin, cellulose derivative, gelatin, collagen, fibrin, polysaccharide, hyaluronic acid, polylactic acid, copolymer of lactic acid with glycolic acid, polyvinylpyrrolidone, dextran andpolyamino acid (*Bioconjugate Chemistry*, 3, 349-362, 1992), modification by lipid such as lecithin (*J. Pharm. Exp. Therap.,* 271, 1672-1677, 1994), modification by copolymer of styrene with maleic acid (SMA), copolymer of divinyl ether with maleic acid anhydride (DIVEMA) and polyvinyl alcohol (PVA) (*BioconjugateChemistry*6, 332-351, 1995), etc. and a preferred one is modification by'polyalkylene glycol.

Specific examples of cytokine modified by polyalkylene glycol are G-CSF modified by polyethylene glycol and a G-CSF derivative ND28 [*J. Biochem.,* 115: 814-819, (1994)].

As the agent of the present invention, it is also possible to use a cytokine having the substantially same activity as above where, in an amino acid sequence constituting the above-mentioned cytokine, one or several amino acid(s) is/are deleted, substituted; inserted or added. The expression "deleted, substituted, inserted or added" means that, in any position of the same sequence, there is deletion, substitution, insertion or addition of one or more amino acid residue(s). Deletion, substitution, insertion or addition as such may take place at the same time and the amino acid residue (s) which is/are deleted, substituted, inserted or added may be either a natural type or a non-natural type. Examples of the natural amino acid residue are L-alanine, L-asparagine, L-aspartic acid, L-glutamine, L-glutamic acid, glycine, L-histidine, L-isoleucine, L-leucine, L-lysine, L-arginine, L-methionine, L-phenylalanine, L-proline, L-serine, L-threonine, L-tryptophan, L-tyrosine, L-valine and L-cysteine.

Examples of amino acid residues which are able to be substituted each other are shown below. Amino acid residues belonging to the same group can be substituted each other. Group A: leucine, isoleucine, norleucine, valine, norvaline, alanine, 2-aminobutanoic acid, methionine, O-methylserine, tert-butylglycine, tert-butylalanine, cyclohexylalanine; group B: aspartic acid, glutamic acid, isoaspartic acid, isoglutamic acid, 2-aminoadipic acid, 2-aminosuberic acid; group C: asparagine, glutamine; group D: lysine, arginine, ornithine, 2,4-diaminobutanoic acid, 2,3-diaminopropionic acid; group E: proline, 3-hydroxyproline, 4-hydroxyproline; group F: serine, threonine, homoserine; group G: phenylalanine, tyrosine. Specifically, ND 28 (*Biochemical and Biophysical Research Communication*, 159: 103-111, 1989) is exemplified as a variant for G-CSF. ND 28 is a substance where the first Thr, the third Leu, the fourth Gly, the fifth Pro and the 17th Cys in G-CSF of a wild type are substituted with Ala, Thr, Tyr, Arg and Ser, respectively.

Examples of hematopoietic stem cell expressing G-CSF receptor are precursor of a myeloid type, lymphocyte, vascular endothelial cell, macrophage, neutrophil, etc. and preferred examples are macrophage and neutrophil.

The cytokine used in the agent of the present invention may be used either solely or jointly.

Among the above-mentioned tissue, bone marrow is a typical one.

Examples of the above multipotential stem cell are CD 45-negative cell, glycophorin A-negative cell and oct 3/4-positive cell, etc. and the preferred multipotential stem cell are CD 45-negative and glycophorin A-negative cell, oct 3/4-positive and CD 45-negative cell, oct 3/4-positive and glycophorin A-negative cell, CD 10-positive and CD 66e-positive cell, CD 13-positive and CD 49b-positive cell, etc., and the more preferred multipotential stem cell are CD 45-negative, glycophorin A-negative and oct 3/4-positive cell, CD 34-positive, CD 117-positive and CD 140-positive cell, CD 45-negative and CD 34-negative cell, CD 45-negative and CD 34-positive cell, etc.

Specific examples are multipotential stem cells which are positive to CD 10, CD 13, CD 49b, CD 49e, CDw 90, Flk 1, EGF-R, TGF-R1, TGF-R2, BMP-R1A, PDGF-R1a and PDGF-R1b and negative to CD 31, CD 34, CD 36, CD 38, CD 45, CD 50, CD 62E, CD 62P, HLA-DR, Muc 18, STRO-1, c-kit, Tie/Tek, CD 44, HLA-class I and 2-microglobulin; multipotential stem cells which are positive to CD 10 and CD 66e and negative to CD 1a, CD 2, CD 3, CD 4, CD 5, CD 7, CD 8, CD 9, CD 11b, CD 11c, CD 13, CD 14, CD 15, CD 16, CD 18, CD 19, CD 20, CD 22, CD 23, CD 24, CD 25, CD 31, CD 33, CD 34, CD 36, CD 38, CD 41, CD 42b, CD 44, CD 45, CD 49d, CD 55, CD 56, CD 57, CD 59, CD 61, CD 62E, CD 65, CD 68, CD 69, CD 71, CD 79, CD 83, CD 90, CD 95, CD 105, CA 117, CD 123, CD 166, glycophorin-A, DRII, Class-1, FLT 3, FMC-7, annexin and LIN; multipotential stem cells which are positive to CD 10, CD 13, CD 34, CD 56, CD 90 andMHC Class-I; multipotential stem cells which are positive to CD 10, CD 13, CD 34, CD 56, CD 90 and MHC Class-1 and negative to CD 1a, CD 2, CD 3, CD 4, CD 5, CD 7, CD 8, CD 9, CD 11b, CD 11c, CD 14, CD 15, CD 16, CD 18, CA 19, CD 20, CD 22, CD 23, CD 24, CD 25, CD 31, CD 33, CD 36, CD 38, CD 41, CD 42b, CD 44, CD 45, CD 49d, CD 55, CD 57, CD 59, CD 61, CD 62E, CD 65, CD 66e, CD 68, CD 69, CD 71, CD 79,, CD 83, CD 95, CD 105, CD 117, CD 123, CD 166, glycophorin-A, DR-II, FLT 3, FMC-7, annexin and LIN; and multipotential stem cells which are positive to CD 34, CD 117, CD 144, CD 140, CD 29 and CD 44 and negative to CD 14, CD 45, Flk-1, CD 31, CD 105, CD 49b, CD 49d, CD 54, CD 102 and CD 106.

There is no particular limitation for the agent of the present invention so far as it contains at least one member selected from the group consisting of the factor which activates granulocyte, monocyte and macrophage, the factor which is secreted from the activated monocyte or macrophage or the factor secreted from a hematopoietic cells expressing the G-CSF receptor, M-CSF, G-CSF, GM-CSF, IL-8 and MMP-9, however, it is preferred to provide as an agent which is manufactured by mixing with one or more pharmacologically acceptable carrier(s) by any method which has been well known in the technical field of pharmaceutical preparations.

Specific examples of the agent are Neu-Up which is a genetically modified G-CSF preparation (manufactured by Kyowa Hakko Kogyo Co., Ltd.), Neutrogin which is a genetically modified G-CSF preparation (manufactured by Chugai Pharmaceutical Co., Ltd.), Gran which is a genetically modified G-CSF preparation (manufactured by Kirin Brewery Co., Ltd.) and Leukoprol which is an M-CSF preparation (manufactured by Kyowa Hakko Kogyo Co., Ltd.).

With regard to the administration route, it is desirable to use the most effective one for the therapy. The preferred are intraoral administration and parenteral administration such as intra-airway, intrarectal, subcutaneous, intramuscular and intravenous administration, and the more preferred are subcutaneous and intravenous administrations.

Sprays, capsules, tablets, granules, syrups, emulsions, suppositories, injections, ointments, tapes, etc. are exemplified as the form for administration.

Examples of the preparation suitable for oral administration are emulsions, syrups, capsules, tablets, powders and granules.

Liquid preparations such as emulsions and syrups can be manufactured using water; saccharide such as sucrose, sorbitol and fructose; glycol such as polyethylene glycol and propylene glycol; oil such as sesame oil, olive oil and soybean oil; antiseptic agent such as p-hydroxybenzoate; flavor such as strawberry flavor and peppermint flavor; etc. as additives.

Capsules, tablets, powders, granules, etc. are manufactured using excipients such as lactose, glucose, sucrose and mannitol; disintegrating agents such as starch and sodium alginate; lubricants such as magnesium stearate and talc; binders such as polyvinyl alcohol, hydroxypropyl cellulose and gelatin; surfactants such as fatty acid ester; plasticizers such as glycerol; etc. as additives.

Examples of the preparations suitable for parenteral administration are injections, suppositories and sprays.

Injections are prepared using a salt solution, a glucose solution, a carrier comprising a mixture of both, etc.

Suppositories are prepared using a carrier such as cacao butter, hydrogenated fat or carboxylic acid.

Sprays are prepared using the compound *per se* or using a carrier or the like which does not irritate mouth and airway mucous membrane of a recipient and disperses the above-mentioned factor as fine particles so as to make the absorption easy.

Specific examples of the carrier are lactose and glycerol. Depending upon the properties of the above-mentioned factor and carrier used, the agent may be prepared as aerosol, dry powder, etc. In those parenteral agents, it is also possible to add a component which is exemplified as an additive for oral agent.

Although the dose of the present pharmaceutical composition varies depending upon age, symptom, etc. of a patient, it is administered to mammals including human being in an amount of 0.1 to 1,000 µg/kg/day in the case of, for example, Neu-Up which is a G-CSF preparation. In the case of Leukoprol which is an M-CSF preparation, 100,000-10,000,000 units/day are administered. The administration is either a single administration for one day only or day after day.

As a result of administration of the agent of the present invention, it is now possible to mobilize the multipotential stem cells to peripheral blood.

### 2. Method for the recovery of multipotential stem cells mobilized to the peripheral blood stem cells

The present invention also relates to a method for the recovery of the multipotential stem cells mobilized to the peripheral stem cells using the agent of the present invention.

An example of the method for the recovery of the multipotential stem cells mobilized to the peripheral blood using the agent of the present invention is a method which comprises selecting mononuclear cells in the peripheral blood by a leukapheresis method and recovering the desired cells by means of recognition using a cell marker.

A leukaphoresis method may be carried out using an instrument such as a CCS (cell collect system) of Haemonetics Corp.

Examples of the method for the recovery of the desired cells by means of recognition using a cell marker are a method which utilized an antibody recognizing the cell marker, a method which utilized microbeads and magnet.

Examples of the desired cells are cells having properties of CD 45-negative and/or glycophorin-A-negative.

Examples of the method using an antibody which recognizes the cell marker are a method which comprises removing CD 45-positive cells and glycophorin-A-positive cells from the mononuclear cells of peripheral blood collected by the above-mentioned method using an antibody which recognizes CD 45 which is a common antigen for lymphocytes, glycophorin-A which is a common antigen for erythroblasts, etc.

An example of the method using microbeads and magnet is a method which comprises incubating microbeads (Miltenyi Biotec) of glycophorin-A and CD 45 with mononuclear cells prepared fromperipheral blood at roomtemperature for 15 minutes and then removing CD 45-positive cells and glycophorin-A-positive cells using Super MACS (Miltenyi Biotec) which is a magnet. About 99.5% of the cells obtained by the present treatment were CD 45-negative and glycophorin-A-negative and, in those cells, multipotential stem cells were contained.

### 3. Method for separation and incubation of the multipotential stem cells mobilized to peripheral blood

As a method for separation and incubation of the multipotential stem cells from the CD 45-negative and glycophorin-A-negative cells prepared in the above 2, there are following two methods.

The first one is as follows. The CD 45-negative and glycophorin-A-negative cells prepared from peripheral blood mononuclear cells are cultured for 2 to 3 weeks on a culture dish coated with 5 ng/ml of fibronectin in a medium (medium-A) using a low-glucose DMEM, MCDB-201 as a fundamental medium and containing 10 µg/ml of insulin, 5.5 µg/ml of transferring 5 ng/ml of Selenium, 0.5 µg/ml of bovine serum albumin, 0.01 µM of dexamethasone, 0.1 mM of L-ascorbic acid 2-phosphate, 2% of fetal calf serum, 10 ng/ml of PDGF, 10 ng/ml of EGF, 10 ng/ml of IGF and 1,000 IU of LIF, and small adhesive cells form a colony. The property of the cells thus prepared can be analyzed using a fluorescence activated cell sorter (FACS) after staining the cells with an antibody which recognizes a cell surface marker.

The cell marker can be identified by subjecting the multipotential stem cells prepared by the above method to an FACS analysis.

As the cell marker for the multipotential stem cells, CD 10, CD 13, CD 49b, CD 49e, CDw 90, Flk 1, EGF-R, TGF-R1, TGF-R2, BMP-R1A, PDGF-R1a, PDGF-R1b, CD 31, CD 34, CD 36, CD 38, CD 45, CD 50, CD 62E, CD 62P, HLA-DR, Muc 18, STRO-1, c-kit, Tie/Tek, CD 44, HLA-class I, 2-microglobulin, etc. can be used.

An example of the multipotential stem cells prepared by the above-mentioned method is a cell which is positive to CD 10, CD 13, CD 49b, CD 49e, CDw 90, Flk 1, EGF-R, TGF-R1, TGF-R2, BMP-R1A, PDGF-R1a and PDGF-R1b and negative to CD 31, CD 34, CD 36, CD 38, CD 45, CD 50, CD 62E, CD 62P, HLA-DR, Muc 18, STRO-1, c-kit, Tie/Tek, CD 44, HLA-class I and 2-microglobulin.

The second method for the preparation of the multipotential stem cells is as follows. The CD 45-negative and glycophorin-A-negative cells obtained from the peripheral bloodmononuclear cells are cultured in an EMEMmedium (medium-B) containing 10% of horse serum and grown until the cells become confluent, and then the cells are recovered using a PBS solution containing 0.05% of trypsin and 0.0744% of EDTA and cooled and frozen down to -80°C in a medium-B containing 7.5% of DMSO. The frozen cells are thawed at 37°C, and the cells are recovered by a centrifugation and cultured again in the medium-B. As a result of repetition of freezing and thawing as such, the multipotential stem cells are concentrated.

In the multipotential stemcells prepared by such amethod, it is possible to identify the cell marker by means of analysis using a flow cytometer.

As the multipotential stem cells prepared by the above-mentioned method, mentioned are (1) cell which is positive to CD 10 and CD 66e and negative to CD 1a, CD 2, CD 3, CD 4, CD 5, CD 7, CD 8, CD 9, CD 11b, CD 11c, CD 13, CD 14, CD 15, CD 16, CD 18, CD 19, CD 20, CD 22, CD 23, CD 24, CD 25, CD 31, CD 33, CD 34, CD 36, CD 38, CD 41, CD 42b, CD 44, CD 45, CD 49d, CD 55, CD 56, CD 57, CD 59, CD 61, CD 62E, CD 65, CD 68, CD 69, CD 71, CD 79, CD 83, CD 90, CD 95, CD 105, CA 117, CD 123, CD 166, glycophorin-A, DRII, Class-I, FLT 3, FMC-7, annexin and LIN; (2) cell which is positive to CD 10, CD 13, CD 34, CD 56, CD 90 and MHC Class-I and negative to CD 1a, CD 2, CD 3, CD 4, CD 5, CD 7, CD 8, CD 9, CD 11b, CD 11c, CD 14, CD 15, CD 16, CD 18, CA 19, CD 20; CD 22, CD 23, CD 24, CD 25, CD 31, CD 33, CD 36, CD 38, CD 41, CD 42b, CD 44, CD 45, CD 49d, CD 55, CD 57, CD 59, CD 61, CD 62E, CD 65, CD 66e, CD 68, CD 69, CD 71, CD 79, CD 83, CD 95, CD 105, CD 117, CD 123, CD 166, glycophorin-A, DR-II , FLT 3, FMC-7, annexin and LIN, (3) cell which is positive to CD 10, CD 13, CD 49b, CD 49e, CDw 90, Flk 1, EGF-R, TGF-R1, TGF-R2, BMP-R1A, PDGF-R1a and PDGF-R1b and negative to CD 31, CD 34, CD 36, CD 38, CD 45, CD 50, CD 62E, CD 62P, HLA-DR, Muc 18, STRO-1, c-kit, Tie/Tek, CD 44, HLA-class I and 2-microglobulin; and (4) cell which is positive to CD 34, CD 117, CD 144, CD 140, CD 29 and CD 44 and negative to CD 14, CD 45, CD 90, Flk-1, CD 31, CD 105, CD 49b, CD 49d, CD 54, CD 102 and CD 106.

The above mentioned cells have a multipotency to differentiate to all tissues except genital gland existing in adults. In addition, the cell which is positive to CD 10, CD 13, CD 34, CD 56, CD 90 and MHC Class-I and negative to CD 1a, CD 2, CD 3, CD 4, CD 5, CD 7, CD 8, CD 9, CD 11b, CD 11c, CD 14, CD 15, CD 16, CD 18, CA 19, CD 20, CD 22, CD 23, CD 24, CD 25, CD 31, CD 33, CD 36, CD 38, CD 41, CD 42b, CD 44, CD 45, CD 49d, CD 55, CD 57, CD 59, CD 61, CD 62E, CD 65, CD 66e, CD 68, CD 69, CD 71, CD 79, CD 83, CD 95, CD 105, CD 117, CD 123, CD 166, glycophorin-A, DR-II, FLT 3, FMC-7, annexin and LIN has a multipotency to mesenchymal system.

Mesenchymal system includes cells derived from mesoderm such as skeletal muscle, smooth muscle, cardiac muscle, bone, cartilage, fat, fibroblast, blood vessel and blood.

As a method for the sorting in FACS, there are a water drop charge method, a cell capture method, etc. ("Flow Cytometer Jiyujizai", pages 14-23, Shujunsha, 1999). In any of those methods, intensity of fluorescence emitted from an antibody bonded to the molecule expressed on the surface of the cell is converted to electric signal, whereby the expressed amount of the antigen can be quantified. It is also possible to separate utilizing a plurality of surface antigens by combination of fluorescence types used therefor. Examples of the fluorescence are FITC (fluorescein isothiocyanate), PE (phycoerythrin), APC (allo-phycocyanin), TR (Texas Red), Cy 3, CyChrome, Red 613, Red 670, PerCP, TRI-Color, Quantum Red, etc. ("Flow Cytometer Jiyujizai", pages 3-13, Shujunsha, 1999).

### 4. Agent for regeneration and treatment of tissues according to the present invention

The agent of mentioned in the above 1 mobilizes the multipotential stem cells to peripheral blood, and damaged tissue can be regenerated by the mobilized multipotential stem cells. Accordingly, the agent of the above 1 can be used for regeneration and treatment of tissues.

As a method for treating the disease using the agent for regeneration and treatment of tissues according to the present invention, mentioned are method which comprises administering the agent to a patient to repair the damaged site, the method which comprises recovering the multipotential stem cells mobilized to the peripheral blood by the agent of the above 1 by the method of the above 2 and transplanting to the damaged site the recovered multipotential stem cells *per se* or after being differentiated to desired cells or tissues *in vitro*, etc.

The agent for regeneration and treatment of tissues according to the present invention can mobilize the multipotential stem cells to peripheral blood, and therefore, the multipotential stem cells are migrated or differentiated to the damaged site to repair the tissue damage.

Thus, for the treatment of the disease where tissue damage quickly takes place, it is preferred to use a method which comprises directing administering to a patient the agent for regeneration and treatment of tissues according to the present invention.

Examples of the disease where tissue damage quickly takes place are irreversible damage of organs or tissues such as cardiac insufficiency, hepatic insufficiency, renal insufficiency, cardiovascular damage, cerebrovascular damage, bone marrow damage, side effect by cancer chemotherapy or cancer immunotherapy, etc.

It is also possible to treat the diseases by administering the agent for regeneration and treatment of tissues according to the present invention recovering the multipotential stem cells mobilized to peripheral blood by the above-mentioned method 2, and transplanting to the damaged site the recovered multipotential stem cells *per se* or after being differentiated to desired cells or tissues *in vitro.*

When the multipotential stem cells or differentiated cells are used for the treatment, they are washed with a physiological saline solution using Hemolite 2 Plus of Haemonetics Corp. or the like. As the instrument, an instrument which is in a completely closed system and which can concentrate, wash and recover the cultured cells is exemplified, and the preferred is an instrument which can remove the substance used for the culture and differentiation such as cytokine to an extent of almost 100%. The recovered multipotential stem cells or the differentiated cells as such can be used for the treatment either by means of infusing into veins by a common instillation or by a direct infusion to the diseased site.

Such a method is preferably used for the treatment of diseases accompanied by chronic tissue damage or degeneration.

Examples of the diseases accompanied by chronic tissue damage or degeneration are neurodegenerative disease, arthritis, refractory inflammatory intestinal disorder, osteoporosis, arteriosclerosis and diabetes mellitus.

According to the present invention, it is also possible that not only the factor which activates granulocyte, monocyte and macrophage but also granulocyte, monocyte and macrophage is directly transplanted to the tissue, whereby the multipotential stem cells are mobilized to the damaged site.

Granulocyte, monocyte and macrophage can be prepared by collection of CD 14-positive cells from peripheral blood mononuclear cells using a magnetic cell sorting system (MACS). They can also be prepared by incubation of CD 34-positive, AC 133-positive or CD 34- and AC 133-double positive hematopoietic stem cells separated from bone marrow or cord blood using Methoculto H4434V (StemCell Tec).

### Best Mode for Carrying Out the Invention

### Example 1. Changes in properties of cells in peripheral blood by a sole administration of G-CSF or a combined administration of G-CSF and M-CSF

### (1) Changes in numbers of cell mobilized to peripheral blood

Changes in numbers of undifferentiated stem cell mobilized to peripheral blood by administration of G-CSF or G-CSF and M-CSF were investigated.

As shown below, group A comprising 4 mice, group B comprising 5 mice and group C comprising 5 mice were prepared, and three different kinds of agents were administered for consecutive five days. Specially, in the mouse group A, 10 µg of G-CSF (manufactured by Kyowa Hakko Kogyo Co., Ltd.; trade name: Neu-Up) was subcutaneously injected to each of four male Balb/c mice (Nippon SLC) of eight weeks age per day for consecutive five days. In the mouse group B, 10 µg of G-CSF (manufactured by Kyowa Hakko Kogyo Co., Ltd.; trade name: Neu-Up) and 10⁵ units of M-CSF (manufactured by Kyowa Hakko Kogyo Co., Ltd.; trade name: Leukoprol) were subcutaneously injected to each of five male Balb/c mice of eight weeks age per day for consecutive five days. In the mouse group C, 200 µl of PBS (phosphate buffered saline) (pH 7.4) (manufactured by Life Technologies) were subcutaneously injected to each of five male Balb/c mice of eight weeks age per day for consecutive five days.

On the next day of the final administration of the agent, mice in each of the groups A, B and C were anesthetized with diethyl ether, and about 1.2 ml of peripheral blood were collected from ophthalmic artery to a tube which was previously charged with 100 units of heparin sodium (manufactured by Takeda Chemical Industries). Then, 0.9% NaCl in the same amount as the collected blood was added thereto for dilution, which was layered on 1.4 ml of Nyco Prep 1.077 Animal (manufactured by DaiichiKagaku Yakuhin), and centrifuged at 600 × g for 30 minutes at room temperature. The layer of the mononuclear cell suspension on the interface was recovered, mixed with 1 ml of PBS and centrifuged at 400 × g for 15 minutes at room temperature. The supernatant was removed, the precipitated mononuclear cells were suspended in 250 µl of an MACS buffer [PBS containing 0.5% of BSA (bovine serum albumin)], and subjected to reaction for 15 minutes on ice with 70 µl of mouse CD 45 microbeads (manufactured by Miltenyi Biotec) and 30 µl of Ter-119 microbeads (manufactured by Miltenyi Biotec). To the reaction was added 4 ml of an MACS buffer, and the mixture was centrifuged at' 300 × g for 10 minutes. Then, the supernatant fluid was removed, and the precipitate was suspended in 400 µl of an MACS buffer. The suspended cells were passed through a separation column MS (manufactured by Miltenyi Biotec) to remove CD 45-positive and Ter 119-positive cells and then CD 45-negative and Ter 119-negative cells contained in the peripheral blood were concentrated. CD 45 is a marker for white blood cells while Ter 119 is a marker for red blood cells of mice. Total numbers of CD 45-negative and Ter 119-negative cells prepared by removal of mature cells such as white blood cells and red blood cells contained in peripheral blood were 2.88 × 10⁴, 2.32 × 10⁴ , 4.72 × 10⁴ and 2.08 × 10⁴, respectively, in the mouse group A (G-CSF being administered); 2.76 × 10⁴, 7.93 × 10³, 3.88 × 10⁴, 4.94 × 10⁴ and 3.07 × 10⁴ , respectively, in the mouse group B (G-CSF and M-CSF being administered); and 5.28 × 10⁵, 5.19 × 10³, 2.60 × 10³, 2.67 × 10³ and 2.67 × 10³, respectively; in the mouse group C (PBS being administered). As compared with the group to which PBS was administered, cell numbers in peripheral blood increased in the groups to which G-CSF was solely administered or G-CSF and M-CSF were administered in combination.

### (2) Analysis of cells mobilized to peripheral blood

Among the cells which were mobilized into the peripheral blood, numbers of cells expressing various kinds of cell surface antigens were analyzed using a FACS Calibur (manufactured by Becton Dickinson). In the analysis, antibody which reacts with each of five antigens - CD 34, c-kit, CD 10, Sca-1 and CD 140a - expressed on the surface of multipotential stem cells was used.

Firstly, each of the CD 45-negative and Ter 119-negative cells of the groups A to C concentrated by the above method was recovered, and the cells in each group were divided into equal three and used for the following analysis (hereinafter, referred to as "divided cells").

At first, the divided cells in each group were centrifuged at 400 × g for 10 minutes at room temperature, suspended in 20 µl of a DMEM medium (manufactured by Life Technologies) containing 10% of serum, then 10 µl of FITC-labeled anti-mouse CD 34 antibody (manufactured by BD PharMingen) and 10 µl of a PE-labeled anti-mouse c-kit antibody (manufactured by BD PharMingen) were added thereto, and allowed to react on ice for 30 minutes with shielding from the light. After washing with a DEME medium, intensities of fluorescence of FITC and PE were tested using an FACS Calibur (manufactured by Becton Dickinson), and numbers of antigen-expressing cells were measured. As a result, numbers of CD 34-positive cells were 46.7 in average in the mouse group A, 35.3 in average in the mouse group B and 9.3 in average in the mouse group C, while the'numbers of c-kit-positive cells were 31.3 in average in the mouse group A, 13.5 in average in the mouse group B and 10.7 in average in the mouse group C.

Then, the divided cells in each group were subjected to reaction with 10 µl of a PE-labeled anti-mouse Sca-1 antibody (manufactured by BD Pharmingen) and 10 µl of a rabbit anti-CD 10 antibody (manufactured by Santa Cruz Biotechnology) on ice for 30 minutes, which was washed with a DMEM medium and subjected to reaction with 10 µl of an FITC-labeled anti-rabbit Ig antibody (manufactured by BD PharMingen), and the numbers of CD 10-positive cells and Sca 1-positive cells were analyzed by measuring intensities of FITC and PE, respectively, using FACS. As a result, the number of CD 10-positive cells were 18.7 in average in the mouse group A, 9.8 in average in the mouse group B and 1.7 in average in the mouse group C.

The numbers of Sca-1-positive cells were 18.7 in average in the mouse group A, 11.8 in average in the mouse group Band 1.7 in average in the mouse group C.

Further the divided cells in each group were subjected to reaction with 10 µl of a rat anti-CD 140a antibody (manufactured by BD PharMingen) on ice for 30 minutes, which was washed with a DMEM medium and subjected to reaction with 10 µl of an FITC-labeled anti-rat Ig antibody (manufactured by BD PharMingen), and numbers of the CD 140a-positive cells were analyzed by measuring the intensity of FITC using FACS. As a result, numbers of CD 140a-positive cells were 19.3 in average in the mouse group A, 6.3 in average in the mouse group B and 4.7 in average in the mouse group C.

The above result shows that G-CSF can mobilize not only hematopoietic stem cell, monocyte and macrophage but also multipotential stem cell to peripheral blood.

### Example 2. Changes in the numbers of CD 45-negative, Ter 119-negative and CD 13-positive cell mobilized to peripheral blood by administration of G-CSF

Group D comprising 5 mice and group E comprising 5 mice were prepared, and two different kinds of agents were administered for consecutive five days as similar to Example 1. Specifically, in the mouse group D, 10 µg of G-CSF (manufactured by Kyowa Hakko Kogyo Co. , Ltd.; trade name: Neu-Up) were subcutaneously injected to each of five male Balb/c mice (Nippon SLC) of eight weeks age per day for consecutive five days. In the mouse group E, 200 µl of PBS (phosphate buffered saline) (pH 7.4) (manufactured by Life Technologies) were subcutaneously injected to each of five male Balb/c mice of eight weeks age per day for consecutive five days.

On the next day of the final administration of the agent, mice in each of the groups D and E were anesthetized with diethyl ether, and about 1.2 ml of peripheral blood were collected from ophthalmic artery to a tube which was previously charged with 100 units of heparin sodium (manufactured by Takeda Chemical Industries, Ltd.). Then, 0.9% Nacl in the same amount as the collected blood was added thereto for dilution, which was layered on 1.4 ml of Nyco Prep 1.077 Animal (manufactured by Daiichi Pure Chemicals Co., Ltd.) and centrifuged at 600 × g for 30 minutes at room temperature. The layer of the mononuclear cell suspension on the interface was recovered, mixed with 1 ml of PBS and centrifuged at 400 × g for 15 minutes at room temperature. The supernatant was removed, and the precipitated mononuclear cells were suspended in 250 µl of an MACS buffer [PBS containing 0.5% of BSA (bovine serum albumin)] and subjected to reaction for 15 minutes on ice with 70 µl of mouse CD 45 microbeads (manufactured by Miltenyi Biotec) and 30 µl of Ter-119 microbeads (manufactured by Miltenyi Biotec). To the reaction solution was added 4 ml of an MACS buffer, and the mixture was centrifuged at 300 × g for 10 minutes. The supernatant was removed, and the precipitate was suspended in 400 µl of an MACS buffer. The suspended cells were passed through a separation column MS (manufactured by Miltenyi Biotec) to remove CD 45-positive and Ter 119-positive cells, and then CD 45-negative and Ter 119-negative cells contained in the peripheral blood were concentrated.

Among the concentrated CD 45-negative and Ter 119-negative cells, numbers of CD 13-expressing cells were analyzed using an FACS Calibur (manufactured by Becton Dickinson) using CD 13 which was confirmed of its expression in the multipotential stem cell surface as an indicator. Firstly, each of the CD 45-negative and Ter 119-negative cells concentrated by the above method were centrifuged at 400 × g for 10 minutes at room temperature, suspended in 20 µl of a DMEM medium (manufactured by Life Technologies) containing 10% of serum, then 10 µl of a PE-labeled anti-mouse CD 13 antibody (manufactured by BD PharMingen) and 10 µl of a Per CP-labeled anti-mouse CD 45 antibody (manufactured by BD PharMingen) were added thereto, and the reaction was carried out on ice for 30 minutes with shielding from the light. After washing with a DMEM medium, intensity of fluorescence was measured using an FACS Calibur (manufactured by Becton Dickinson), and numbers of antigen-expressing cells were analyzed. As a result, cell numbers of CD 45-negative, Ter 119-negative and CD 13-positive mononuclear cell were 29.5 in average in the mouse group D and 11.9 in average in the mouse group E. The result shows that G-CSF can mobilize not only hematopoietic stem cell, monocyte and macrophage but also multipotential stem cell to peripheral blood.

### Example 3. Property of cells mobilized to peripheral blood by administration of G-CSF

### (1) Transplantation of cells mobilized to peripheral blood mobilized by administration of G-CSF to mice irradiated with X-ray

The property of cells mobilized to peripheral blood by administration of G-CSF was analyzed by transplanting the cells to mice.

Mice (F4 of C57BL/6 × 129 strain) of ten weeks age, in which GFP gene was integrated into all the cells in the body and GFP protein was expressed, were divided into a group F comprising 3 mice'and a group G · comprising 1 mouse, and the following agents were administered' to each of them. Specifically, to the group F, 10 µg per day of G-CSF (manufactured by Kyowa Hakko Kogyo Co.; Ltd.; trade name: Neu-Up) was subcutaneously injected to each mouse for consecutive five days. To the group G, 200 µl per day of PBS (phosphate buffered saline) (pH 7.4) (manufactured by Life Technologies) was subcutaneously injected to each mouse for consecutive five days.

On the next day of the final administration of the agent, each mouse of the groups F and G was anesthetized with diethyl ether, and peripheral blood was collected from ophthalmic vein, recovered in a tube in which heparin sodium (manufactured by Takeda Chemical Industries, Ltd.) was previously charged, and the recovered peripheral blood was passed through a 100-µm cell strainer (manufactured by Becton Dickinson). With regard to the group G, femur was excised, muscles attached to the femur were cut off using scissors so that whole femur was exposed, and then both ends were cut by scissors, front end of injection needle being attached with a needle of 27G manufactured by Thermo and containing PBS was inserted into the cut end of the knee joint side of the femur, and bone marrow cells were collected by blowing the PBS into a test tube, and the collected bone marrow cells were passed through a 100-µm cell strainer (manufactured by Becton Dickinson).

The peripheral blood or the bone marrow thus collected was transplanted by infusing into C57BL/6 mice from tail vein as follows.

Firstly, the C57BL/6 mice of eight weeks age (Nippon Claire) were prepared, and on the day before the injection from tail vein, they were previously irradiated with X-ray in a dose of 12 Gy using an X-ray irradiating apparatus (manufactured by Hitachi Medico). Those mice were divided into groups H, I, J and K. To each mouse in the group H were transplanted to its tail vein 300 µl of peripheral blood derived from the mouse in group F; to each mouse in the group I were transplanted to its tail vein 300 µl per mouse of peripheral blood derived from the mouse in group G; to each mouse in group J were transplanted to its tail vein 3.0 × 10⁶ of bone marrow cells derived from the mouse in group G; and the mouse in group K were not subjected to transplantation.

As a result, in the groups I and K, all the mouse were dead within 7 to 10 days from the transplantation while, in the groups H and J, the mice were alive even after eight weeks from the transplantation. However, in some of mice of the group J in which bone marrow was transplanted, abnormalities. in appearance and behavior were noted, for example, hair came out and skin was sore and they walked with inclined head and walking was unnatural. In the group H in which peripheral blood of mice to which G-CSF was administered was transplanted, no abnormality in appearance and behavior was noted.

The result shows that stem cells having an ability of differentiating into tissues such as skin are mobilized to peripheral blood of mice by the administration of G-CSF.

### (2) Dissection of mice to which cells were transplanted

The mice of the group H obtained in (1) were dissected and subjected to perfusion and fixation, and each organ was excised.

Specifically, the mouse was anesthetized by intraperitoneal injection of Nembutal (manufactured by Dainippon Pharmaceutical Co., Ltd.), total body was wetted by 70% ethanol, skin of thigh was picked up, the area from knee to root of thigh was cut and opened by scissors to expose thigh artery and vein. They were ligatured by suture made of silk (manufactured by Natsume Seisakusho Co., Ltd.), and the thigh was cut from the end of the ligatured site. Shinbone was excised therefrom, muscle attached to the shinbone was removed by scissors to expose total shinbone, both ends thereof were cut with scissors, front end of injection needle being attached with a needle of 23G manufactured by Thermo and containing PBS was inserted into the cut end of the knee joint side of the shinbone, and bone marrow cells were collected by blowing the PBS into a test tube.

On the other hand, the mouse was subjected to laparotomy and thoracotomy to expose the heart, 25G of a needle for intravenous injection equipped with a wing manufactured by Thermo was inserted into left ventricle, right auricle was cut off, and 20 ml of PBS were flowed and perfused through the whole body. Blood overflowed from the heart at that time was collected, as a peripheral blood, in a tube in which 100 units of heparin sodium (manufactured by Takeda Chemical Industries, Ltd.) were charged.

After all PBS was flowed, 20 ml of a fixing solution [4% PFA (paraformaldehyde), PBS] were flowed by the same operation to fix.

Then, the lung was excised together with airway, a 20-ml syringe equipped with a catheter attached to a surf low indwelling needle (20 G) manufactured by Thermo containing an OCT (optimum cutting temperature) compound (manufactured by Miles) diluted to two-fold with PBS was inserted into the airway, the front end of catheter and the airway were bonded using a suture made of silk (manufactured by Natsume Seisakusho Co., Ltd.), and 10 ml of the OCT solution diluted with PBS to two-fold were infused into lung through the airway. After the infusion, the lung was cut into blocks of several mm square, embedded with an OCT compound and frozen with isopentane which was cooled with dry ice.

Other organs were also excised from the mouse, immersed in a fixing solution [4% PFA (paraformaldehyde), PBS] at 4°C for 2 hours, rinsed with PBS, immersed in a high sucrose solution [20% sucrose, PBS], allowed to stand at 4°C over night, cut into blocks of several mm square on the next day, embedded with an OCT compound and frozen with isopentane which was cooled with dry ice.

The tissue thus frozen was sliced into the thickness of 10 µm using a cryostat, adhered to a slide glass coated with APS (manufactured by Matsunami) and well dried to prepare frozen sections.

The peripheral blood cells prepared during the above-mentioned operation were diluted with the same amount of 0.9% NaCl, which was layered on 1.4 ml of Nyco Prep 1.077 Animal (manufactured by Daiichi Pure Chemicals Co., Ltd. ) and centrifuged at room temperature for 30 minutes at 600 × g. A layer of the mononuclear cell suspension of the interface was recovered, mixed with 1 ml of PBS and centrifuged at room temperature for 15 minutes at 400 × g. The supernatant was removed, the precipitated mononuclear cells were suspended in 0.5 ml of PBS, and.the rate of numbers of GFP-positive cells among the peripheral mononuclear cells was measured using a FACS Calibur (manufactured by Becton Dickinson). As a result, the rate of the GFP-positive cells was 90.3%.

As to bone marrow cells, rate of the GFP-positive cells was also measured using a FACS Calibur in the same manner, and 87.3% were occupied by the GFP-positive cells.

Frozen section prepared from each organ by excision was observed under Axiophot 2, a fluorescence microscope manufactured by Zeiss, and it was confirmed that many GFP-positive cells were present in nearly all organs of the body such as lung, heart, liver, brain, stomach, skin, small intestine, large intestine, skeletal muscle, pancreas, spleen, kidney and trachea. Especially in the brain, many GFP-positive cells were observed in the areas such as olfactory bulb and choroid plexus. The result shows that stem cells mobilized to peripheral blood of mouse by administration of G-CSF functions for repair of various tissues of the body.

### (3) Identification of the property of GFP-positive cells by immunostaining

The frozen sections prepared in (2) were stained with various antibodies as follows to investigate the property of the GFP-positive cells.

Firstly, tissue sections of various organs were subjected to immunostaining using an anti-cytokeratin antibody. Cytokeratin is a marker for epithelial cells.

The frozen section prepared by excision of the skin was put on a slide glass, and the slide glass was immersed in PBS for 5 minutes for three times. After washing the slide glass, it was immersed for 15 minutes in Proteinase K (manufactured by Gibco BRL) diluted with PBS so as to make the final concentration 10 mg/ml. After washing with PBS once, it was subjected to reaction with a fixing solution [4% PFA (paraformaldehyde), PBS] at room temperature for 15 minutes. After washing twice with PBS, it was immersed in a blocking solution [10% porcine serum (manufactured by Dako), PBS] at room temperature for 1 hour, and subjected to reaction at room temperature for 1 hour with a solution in which a primary antibody [Monoclonal Mouse Anti-Human Cytokeratin, Clones AE1/AE3] (manufactured by Dako) was diluted to 50-foldwith PBS containing 1.5% of porcine serum. After washing with PBS for four times, it was subjected to reaction at room temperature for 1 hour with a solution in which the second antibody [Cy3-conjugated Affini Pure Goat Anti-Mouse IgG (H + L)] (manufacture by Seikagaku Corporation) was diluted to 800-fold with PBS containing 1.5% of porcine serum. After washing with PBS for four times more, Vectashield Mounting Medium with DAPI (manufactured by vector Laboratories) was dropped into the section, sealed with a cover glass and observed under a fluorescence microscope Axiophot 2 manufactured by Zeiss. Since GFP-positive and cytokeratin-positive cells were observed, which shows that, in the cells of peripheral blood mobilized by G-CSF used for the transplantation, there were contained stem cells having an ability of being taken to the skin and differentiating into epithelial cells.

Frozen section of large intestine was similarly subjected to the staining using an anti-cytokeratin antibody and observed under a fluorescence microscope, and as a result, GFP-positive and cytokeratin-positive cells were observed. The result shows that, in the cells of peripheral blood mobilized by G-CSF used for the transplantation, there were contained stem cells having an ability of being taken to the large intestine and differentiating into epithelial cells.

Frozen section of small intestine was similarly subjected to the staining using an anti-cytokeratin antibody and an antibody to CD 45 which is a marker for blood cells. When it was observed under a fluorescence microscope, there were observed GFP-positive and CD 45-negative cells. The result shows that, in the cells of peripheral blood mobilized by G-CSF used for the transplantation, there were contained stem cells having an ability of being taken to the small intestine and differentiating into cells which are other than blood cells.

Then, various organs were subjected to immunostaining using an anti-CD 45 antibody.

A slide glass on which frozen section prepared by excision of lung was washed by immersion for 5 minutes into PBS for three times, the slide glass was immersed at room temperature for 10 minutes in a Dako Biotin Blocking System (1) solution (manufactured by Dako), washed twice with PBS, immersed at room temperature for 10 minutes into a Dako Biotin Blocking System (2) solution (manufactured by Dako) and washed twice with PBS again.

Then, it was immersed at room temperature for 1 hour in a blocking solution [10% porcine serum (manufactured by Dako), PBS] and then subjected to reaction at room temperature for 1 hour with a solution in which the primary antibody [Biotin anti-mouse CD 45 (LCA, Ly 5)] (manufactured by BD Pharmingen) was diluted to 100-fold with PBS containing 1.5% porcine serum. After washing with PBS for 4 times, it was subjected to reaction at room temperature for 1 hour with a solution in which the second antibody (streptavidin, Alexa Fluor 594 conjugate) (manufactured by Molecular Probe) was diluted with PBS containing 1.5% of porcine serum to the final concentration 5 µg/ml . After washing with PBS for 4 times more, a Vectashield Mounting Medium with DAPI (manufactured by Vector Laboratories) was dropped on the section, sealed with a cover glass and observed under a fluorescence microscope. As a result, GFP-positive and CD 45-negative cells were observed. The result shows that, in the cells of peripheral blood mobilized by G-CSF used for the transplantation, there were contained stem cells having an ability of being taken to the lung and differentiating into cells other than blood cells.

Frozen section of liver was similarly subjected to the staining using an anti-CD 45 antibody, and observed under a fluorescence microscope, and GFP-positive and CD 45-negative cells were observed. The result shows that, in the cells of peripheral blood mobilized by G-CSF used for the transplantation, there were contained stem cells having an ability of being taken to the liver and differentiating into cells other than blood cells.

Frozen sections of heart, brain, stomach, skin, small intestine, large intestine, skeletal muscle and pancreas were similarly subjectedto the staining using an anti-CD 45 antibody. When they were observed under a fluorescence microscope, there were observed GFP-positive and CD 45-negative cells in each ofthetissues. The result shows that, in the cells of peripheral blood mobilized by G-CSF used for the transplantation, there were contained stem cells having an ability of being taken to heart, brain, stomach, skin, small intestine, large intestine, skeletal muscle and pancreas and differentiating into cells other than blood cells.

### Industrial Applicability

The present invention relates to an agent which mobilizes multipotential stem cells having an ability of differentiating into various tissues to peripheral blood, an agent for the treatment by tissue regeneration containing the agent, a method for recovering of multipotential stem cells using the agent, and a method for inducing differentiation of the multipotential cells prepared by the above method to somatic cells.

## Claims

1. An agent mobilizing the multipotential stem cells from tissues to peripheral blood which contains, as an active ingredient, a cytokine selected from the group consisting of a cytokine which activates monocyte or macrophage, a cytokine which is secreted from the activated monocyte or macrophage and a cytokine which is secreted from hematopoietic cells where G-CSF receptor is expressed.

2. The agent according to claim 1, wherein the cytokine is a cytokine which is selected from the group consisting of G-CSF, M-CSF, GM-CSF and IL-8.

3. The agent according to claim 1, wherein the cytokine which activates monocyte or macrophage is selected from the group consisting of M-CSF and GM-CSF.

4. The agent according to claim 1, wherein the hematopoietic cells expressing the G-CSF receptor is granulocyte or neutrophil.

5. The agent according to claim 4, wherein the cytokine secreted from neutrophil is IL-8.

6. The agent according to any of claims 1 to 5, wherein the cytokine is a chemically modified one.

7. The agent according to claim 6, wherein the cytokine is chemically modified with polyalkylene glycol.

8. The agent according to any of claims 1 to 7, wherein one or several amino acid(s) in the amino acid sequence constituting the cytokine is/are deleted, substituted, inserted or added and the cytokine has the substantially same activity.

9. The agent according to claim 1, wherein the tissue is bone marrow.

10. The agent according to claim 1, wherein the multipotential stem cell is selected from the group consisting of CD 45-negative cell, glycophorin A-negative cell and oct 3/4-positive cell.

11. The agent according to claim 1, wherein the multipotential stem cell is selected from the group consisting of CD 45-negative and glycophorin A-negative cell, oct 3/4-positive and CD 45-negative cell, oct 3/4-positive and glycophorin A-negative cell, CD 45-negative, glycophorin A-negative and oct 3/4-positive cell and CD 45-negative and CD 34-negative cell.

12. The agent according to claim 1, wherein the multipotential stem cell is selected from the group consisting of CD 34-positive, CD 117-positive and CD 140-positive cell, CD 10-positive and CD 66e-positive cell, CD 13-positive and CD 49b-positive cell and CD 45-negative and CD 34-positive cell.

13. A method for the recovery of the multipotential stem cells mobilized to the peripheral blood stem cells, wherein the agent mentioned in any of claims 1 to 12 is used.

14. A method for regeneration of tissues, wherein the multipotential stem cells obtained by the method mentioned in claim 13 is used.

15. A method for inducing the differentiation of the multipotential stem cells mobilized to the peripheral blood stem cells to somatic cells using the agent mentioned in any of claims 1 to 12.

16. The method according to claim 15, wherein the somatic cells are those selected from the group consisting of red blood cells, white blood cells, platelets, fibroblasts, adipocytes, skeletal muscle cells, smoothmuscle cells, cardiac muscle cells, neurons, glias, oligodendrocytes, vascular endothelial cells, epidermal cells of skin, dermal cells of skin, hair follicle cells, osteocytes, osteoblasts, osteoclasts, chondrocytes, epithelial cells of trachea, alveolar cells, epithelial cells of gastrointestenal tract, epithelial cells of mouth, ameloblasts, odontoblasts, hepatocytes, Kupffer's cells, epithelial cells of gallbladder, endocrine cells of pancreas, exocrine cells of pancreas, tubular cells of kidney, renal glomeruli cells, epithelial cells of ureter, epithelial cells of urinary tract, endocrine cells of pituitary gland, thyroid cells, adrenocortical cells, adrenomedullary cells, prostatic cells, mammary gland cells, visual cells, pigment epithelial cells, corneal cells, lacrimal cells and tympanic cells.

17. A tissue regeneration therapeutic agent which contains, as an active ingredient, a cytokine selected from the group consisting of a cytokine which activates monocyte or macrophage, a cytokine which is secreted from the activated monocyte or macrophage and a cytokine which is secreted from hematopoietic cells where G-CSF receptor is expressed.

18. The therapeutic agent according to claim 17, wherein the cytokine is selected from the group consisting of G-CSF, M-CSF, GM-CSF and IL-8.

19. The therapeutic agent according to claim 17, wherein the cytokine which activates monocyte or macrophage is selected from the group consisting of M-CSF and GM-CSF.

20. The therapeutic agent according to claim 17, wherein the hematopoietic cells expressing the G-CSF receptor is granulocyte or neutrophil.

21. The therapeutic agent according to claim 20, wherein the cytokine secreted from neutrophil is IL-8.

22. The therapeutic agent according to any of claims 17 to 21, wherein the cytokine is a chemically modified one.

23. The therapeutic agent according to claim 22, wherein the cytokine is chemically modified with polyalkylene glycol.

24. The therapeutic agent according to any of claims 17 to 23, wherein one or several amino acid(s) in the amino acid sequence constituting the cytokine is/are deleted, substituted, inserted or added and the cytokine has the substantially same activity.

25. The therapeutic agent according to any of claims 17 to 24, wherein the tissue is selected from the group consisting of neural tissue, skin tissue, cardiovascular tissue, respiratory tissue; skeletal tissue, connective tissue, blood, immunological tissue, enteric tissue, oral tissue, hepatic tissue, pancreatic tissue, urinary tissue, endocrine gland and sensory tissue.

26. The therapeutic agent according to claim 25, wherein the neural tissue is selected from the group consisting of central nervous system and peripheral nervous system.

27. The therapeutic agent according to claim 25, wherein the skin tissue is selected from the group consisting of epidermis of skin, dermis of skin and hypodermal tissue.

28. The therapeutic agent according to claim 25, wherein the cardiovascular tissue is selected from the group consisting of heart, blood vessel of artery, bloodvessel of vein, capillary vessel and lymph vessel.

29. The therapeutic agent according to claim 25, wherein the respiratory tissue is selected from the group consisting of viscous membrane of nose, airway and air cell.

30. The therapeutic agent according to claim 25, wherein the skeletal tissue is selected from the group consisting of bone, cartilage, joint, ligament and tendon.

31. The therapeutic agent according to claim 25, wherein the connective tissue is selected from the group consisting of fat tissue and fiber tissue.

32. The therapeutic agent according to claim 25, wherein the blood tissue is selected from the group consisting of red blood cell, white blood cell and platelet.

33. The therapeutic agent according to claim 25; wherein the immunological tissue is selected from the group consisting of lymphocyte, monocyte, granulocyte, thymus, lymph node and spleen.

34. The therapeutic agent according to claim 25, wherein the enteric tissue is selected from the group consisting of salivary gland, stomach, duodenum, small intestine, large intestine, rectum and anus.

35. The therapeutic agent according to claim 25, wherein the oral tissue is selected from the group consisting of mucous membrane of mouth, tooth and tongue.

36. The therapeutic agent according to claim 25, wherein the hepatic tissue is selected from the group consisting of liver and gallbladder.

37. The therapeutic agent according to claim 25, wherein the pancreatic tissue is selected from the group consisting of exocrine gland of pancreas and endocrine gland of pancreas.

38. The therapeutic agent according to claim 25, wherein the urinary tissue is selected from the group consisting of renal nephron, urinary tubule of kidney, renal medulla, ureter, bladder and urinary tract.

39. The therapeutic agent according to claim 25, wherein the endocrine gland is selected from the group consisting of pituitary gland, thyroid gland and peripheral nerve.

40. The therapeutic agent according to claim 25, wherein the sensory tissue is selected from the group consisting of gustatory bud, olfactory epithelium, retina, cornea, crystalline lens, labyrinth and peripheral nerve system.
